# EUROPÄISCHE PATENTSCHRIFT

(11) **EP 0 893 097 B1**
(45) Veröffentlichungstag und Bekanntmachung des Hinweises auf die Patenterteilung: **01.09.2004**
(21) Anmeldenummer: 98113307.7
(22) Anmeldetag: 16.07.1998
(51) Int. Cl.: A61B 17/00, A61B 17/16

(54) **Halter für ein chirurgisches Instrument**
Surgical instrument holder
Dispositif support pour instrument chirurgical

(30) Priorität: 23.07.1997 DE 19731522
(43) Veröffentlichungstag der Anmeldung: 27.01.1999
(73) Patentinhaber: ESKA Implants GmbH & Co., 23556 Lübeck (DE)
(72) Erfinder: Schumacher, Erhard Winfried, 65375 Östrich-Winkel (DE)
(74) Vertreter: Fuchs Mehler Weiss & Fritzsche

(56) Entgegenhaltungen:
- DE-A- 3 934 610
- US-A- 4 319 577
- US-A- 5 171 312
- US-A- 5 222 956

## Beschreibung

Die vorliegende Erfindung betrifft eine Kombination eines Halters für ein chirurgisches Instrument mit einem chirurgischen Instrument,wobei der Halter mit dem chirurgischen Instrument durch einen Schnellverschluss koppelbar ist, gemäß dem Oberbegriff des Anspruchs 1.

Gattungsgemäße Halter sind beispielsweise aus der DE 39 34 610 A1 bekannt. Denkt man beispielsweise an eine Raffelfräserklinge als chirurgisches Instrument, mit der das natürliche Acetabulum zur Vorbereitung der Implantation eines Metallsockels eines künstlichen Hüftgelenkes präpariert wird, so ist beispielsweise auch ein Bajonettverschluss bekannt, wobei Stege in der Äquatorialebene der Klinge durch den Bajonettverschluss beim Verriegeln umfasst werden. Der Verschluss ist federvorgespannt, so dass der Verschluss stets nur gegen die Federkraft geöffnet werden kann. Gerade bei der Vorbereitung des Acetabulums für die Implantation eines Metallsockels eines künstlichen Hüftgelenkes ist der Wechsel zwischen verschiedenen Größen der Raffelfräserklinge notwendig. Normalerweise wird mit der kleinsten Klinge mit dem Ausfräsen begonnen und die Größe stufenweise, beispielsweise in sieben Stufen, bis zum Maximum erhöht. Dies bedeutet einerseits, dass die Ankoppelung und die Entkoppelung des Halters vom Instrument für den Operateur leicht vonstatten gehen muss. Darüber hinaus aber muss das Instrument und der Halter nach der Operation gereinigt werden. Hierzu erscheint es bei dem bekannten Gerät als nachteilig, dass es sich nicht ohne weiteres in seine Einzelteile zerlegen lässt, die dann relativ unproblematisch gereinigt werden können.

Ein anderer Vorschlag sieht vor, dass der Halter an seinem Koppelungsende mit einer relativ großen Grundplatte versehen ist, auf die entsprechend ausgebildete Instrumente gesetzt werden können. Eine einfache Rastvorrichtung soll die Klinge auf der Grundplatte arretieren. Hierbei stellt es sich als problematisch heraus, dass der Kraftschluss und Formenschluss zwischen Halter und Klinge nicht groß genug ist, um den hohen auftretenden Belastungen beim Ausfräsen des Acetabulums auf Dauer standhalten zu können.

Weitere Halter für chirurgische Instrumente sind im Übrigen bekannt aus der US 5 222 956 A, US 5 171 312 A, EP 0 642 770 A2.

Vor dem aufgezeigten Hintergrund ist es nun die Aufgabe der vorliegenden Erfindung, eine eingangs erwähnte Kombination so weiterzubilden, dass der Halter und das Instrument leicht miteinander verbunden und auch leicht wieder voneinander getrennt werden können.

Gelöst wird diese Aufgabe dadurch, dass die Hinterschnitte von einen Hohlraum im Instrument übergreifenden Stegen als Bestandteil eines Doppel-Kreuzstegess gebildet sind.

Das Instrument weist als Koppelelement über einen Hohlraum gespannte, die Hinterschneidung bildende Stege auf. Insbesondere wenn das Instrument eine Raffelfräserklinge ist, wird der Hohlraum gebildet durch die halbkugelförmige Innenkalotte des Fräsers. Die Stege sind dann in etwa in der Äquatorialebene gespannt.

Die entsprechend ausgebildete Raffeifräserklinge als Instrument in der vorbenannten Kombination ist vorzugsweise so weitergebildet, dass der Steg ein Doppelkreuzsteg in der Äquatorialebene ist. Durch die Mittenöffnung des Doppelkreuzsteges wird der Halter beim Ankoppeln des Instrumentes geführt und das Betätigungsorgan wird währenddessen so betätigt, dass der Dorn im limeren der Hülse nicht zwischen die Kugeln greift. In der Endposition des distalen Halterendes wird dann das Betätigungsorgan freigegeben, woraufhin die erwähnte Feder dafür sorgt, daß der Dorn in Richtung auf den Kugelkäfig gedrückt wird, um zwischen die Kugeln zu greifen und zu arretieren derart, daß die Kugeln den Halter aufgrund des Hintergreifens der Hinterschneidungen am Doppelkreuzsteg sicher arretieren.

Gemäß einer bevorzugten Ausführungsform ist vorgesehen, daß der Halter im wesentlichen aus einer Hülse besteht, in deren Inneren ein Dorn axial verschieblich gelagert ist, wobei der Dorn in vorgeschobener Lage die Kugeln in einem am distalen Ende des Halters vorgesehenen Käfiggehäuse arretiert. Zu diesem Zwecke fährt der Dorn an seinem distalen Ende zwischen die Kugeln und drückt sie auseinander, so daß die Kugeln über die Konturen des Käfiggehäuses teilweise herausragen, um so hinter die Hinterschneidungen am Instrument zu greifen. Darüberhinaus gibt der Dorn die Kugeln in seiner zurückgezogenen Lage frei, damit diese in das Innere des Käfiggehäuses fallen oder gedrückt werden können, wenn die Kugeln durch die Verengung an den Hinterschnitten aus dem Instrument herausgeführt werden. Der Dorn im Inneren der Hülse muß also mittels eines Betätigungsorgans in axialer Richtung so weit verschoben werden können, daß er sich bei Arretierung der Kugeln mit seinem distalen Ende zwischen die Kugeln schiebt und andererseits in die andere Richtung geschoben werden kann, so daß der Dorn nicht mehr zwischen die Kugeln greift.

Vorzugsweise ist der Halter in Form einer Hülse am rückseitigen Ende mittels einer Buchse verschließbar, indem die Buchse vorzugsweise ein Außengewinde und die Hülse ein entsprechendes Innengewinde aufweist. Diese Anordnung läßt sich in einfacher Weise wieder auseinander nehmen, wenn nämlich die Buchse nach außen gewandt ein Innensechskantgewinde für einen sogenannten AO-Schlüssel aufweist, welcher im medizinischen Bereich weit verbreitet ist.

Bevorzugt wird eine Weiterbildung, bei der zwischen dem Dorn und der Buchse eine Spiralfeder eingesetzt ist, die den Dorn in Richtung auf die vorgeschobene Lage vorspannt. Wenn also die Hülse mit der Buchse verschlossen ist, drückt die Feder den Dorn in Richtung auf die Kugeln mit der Neigung, zwischen sie zu fahren und so zu arretieren. Dies hat für die Praxis den Vorteil, daß der Operateur bei Nichtbetätigung eines Betätigungsorganes sicher sein kann, daß das Instrument am distalen Ende des Halters arretiert ist.

Als Betätigungsorgan ist vorzugsweise ein die Hülse durch ein Langloch hindurchgreifender Gewindebolzen vorgesehen, der mit einer entsprechenden Gewindebohrung im Dorn verschraubbar ist. Auch der Gewindebolzen kann nach außen hin wieder einen Innensechskant für einen AO-Schlüssel aufweisen.

In einfacher Weise kann der erfindungsgemäße Halter nach Gebrauch zerlegt werden, nämlich indem beispielsweise zunächst der Gewindebolzen entfernt wird, die Mimik im Inneren der Hülse, also der Dorn, die Feder und die Buchse herausgenommen werden und schließlich die Kugeln durch die Hülse nach außen hin befördert werden. Die Einzelteile können sodann gereinigt und in umgekehrter Reihenfolge in ebenfalls einfacher Weise wieder zusammengefügt werden.

Die Erfindung wird anhand eines Ausführungsbeispieles näher erläutert. Hierbei zeigt:
- Figur 1: den Halter mit angekoppeltem chirurgischen Instrument, im Schnitt,

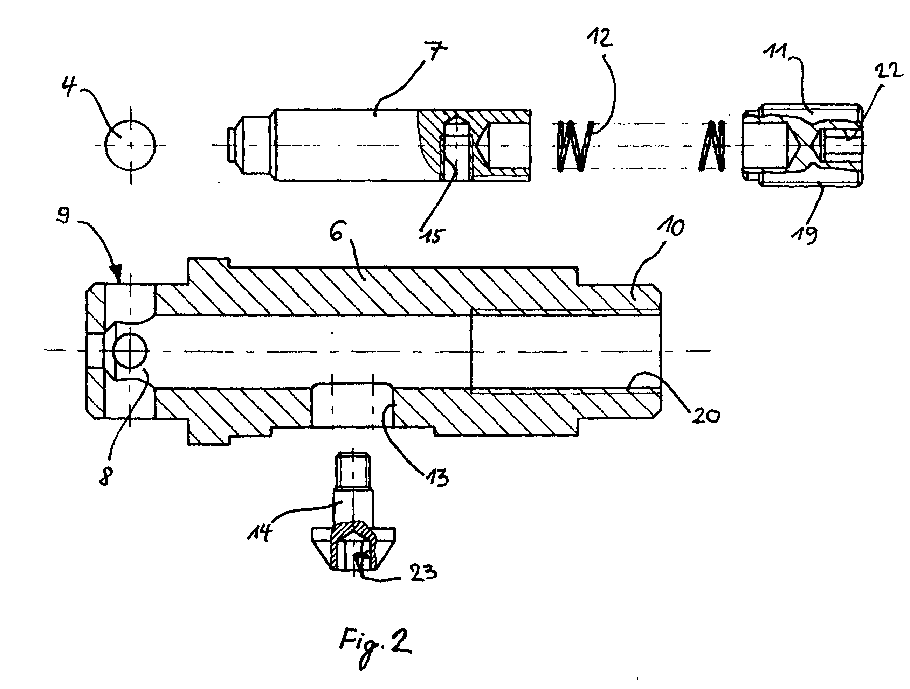

- Figur 2: die Einzelteile des Halters, und
- Figur 3: die Aufsicht auf die Unterseite auf ein chirurgisches Instrument, das mit dem Halter aus den Figuren 1 und 2 koppelbar ist.

Nachfolgend sind gleiche Teile mit denselben Bezugszeichen versehen.

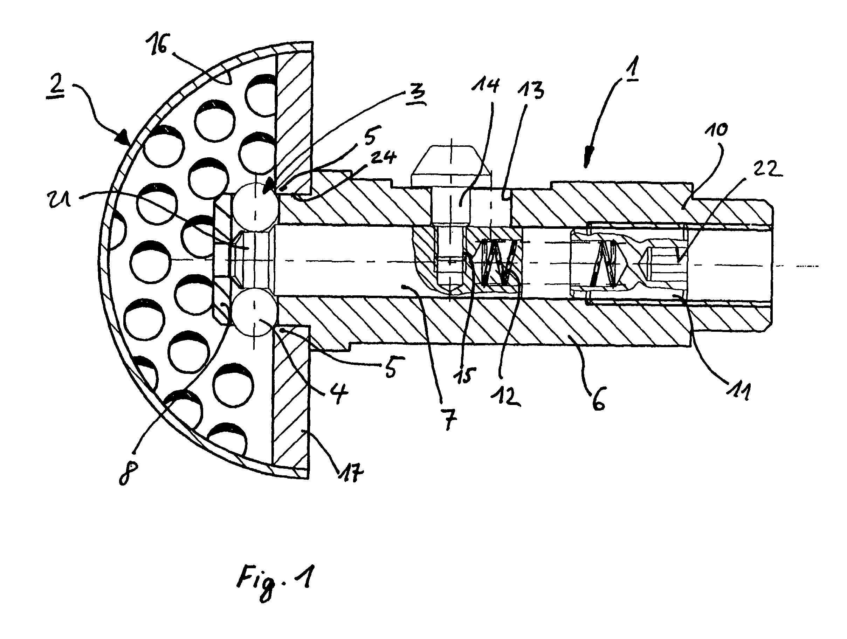

Vorliegend besteht der Halter 1 aus einer Hülse 6. In deren Innerem ist ein Dorn 7 axial verschieblich gelagert. Die Betätigung des Dorns 7 erfolgt vorliegend durch ein Betätigungsorgan in Form eines Gewindebolzens 14, der durch ein Langloch 13 in der Hülse 6 greift und mit der Gewindebohrung 15 im Dorn 6 verschraubt ist. Die Bewegung des Gewindebolzens 14 in Figur 1 nach rechts bewirkt, daß das distale Ende 21 des Doms 7 außer Eingriff kommt mit den Kugeln 4, die einen wesentlichen Bestandteil des Schnellverschlusses 3 bilden. Am distalen Ende 9 der Hülse 6 ist das Käfiggehäuse 8 angeformt, in dem die Kugeln 4 gefangen sind.

Am anderen Ende ist die Hülse 6 durch einen in das Innere der Hülse 6 geschraubten Bolzen 11 verschlossen. Zwischen dem Bolzen 11 und dem Dorn 7 ist vorliegend eine Spiralfeder 12 eingelegt, die den Dorn 7 in Richtung auf das Käfiggehäuse 8 vorspannt. Läßt die Bedienerperson nun den Gewindebolzen 14 wieder los, so sorgt die Spannung der Feder 12 dafür, daß der Gewindebolzen 14 mit dem Dorn 7 in Figur 1 nach links verschoben werden, so daß sich das distale Ende 21 des Dorns 7 zwischen die Kugeln 4 drückt und diese so versucht, aus dem Kugelgehäuse 8 zu verdrängen. Dies gelingt jedoch aufgrund der Käfigsituation nicht. Vielmehr werden die Kugeln 4 in der Stellung arretiert. Hierdurch greifen sie dauerhaft hinter die Hinterschneidungen 5 am Instrument 2.

Die Hinterschneidungen 5 sind gebildet durch Stege 17 in der Äquatorialebene des als Raffelfräserklinge ausgebildeten Instrumentes 2. Die Stege greifen hier über den Hohlraum 16 des Instrumentes.

Die Bedienerperson braucht zum Wechsel des Instrumentes lediglich entgegen der Kraft der Feder 12 den Gewindebolzen 14 in Figur 1 nach rechts zu drücken, wonach der Halter 1 problemlos vom Instrument 2 abziehbar ist.

Zur Demontage des Halters zur Reinigungszwecken ist es vorliegend lediglich notwendig, zunächst die mit einem Außengewinde 19 versehene Buchse 11 aus der Bohrung mit dem Innengewinde 20 in der Hülse 6 herauszuschrauben. Hierzu dient in der Buchse 11 ein übliches Innensechskantgewinde 22 für einen sogenannten AO-Schlüssel. Nach dem Herausschrauben der Buchse 11 kann die Feder 12 entfernt werden. Der Gewindebolzen 14 weist ebenfalls ein Innensechskantgewinde 23 für den AO-Schlüssel auf. Nach Entfernung des Gewindebolzens 14 kann der Dorn aus dem Inneren der Hülse 6 entfernt werden. Schließlich können die Kugeln 4 durch das Innere der Hülse 6 aus dem rückseitigen Ende 10 der Hülse 6 entfernt werden. Die Montage nach der Reinigung erfolgt in einfacher Weise in umgekehrter Reihenfolge.

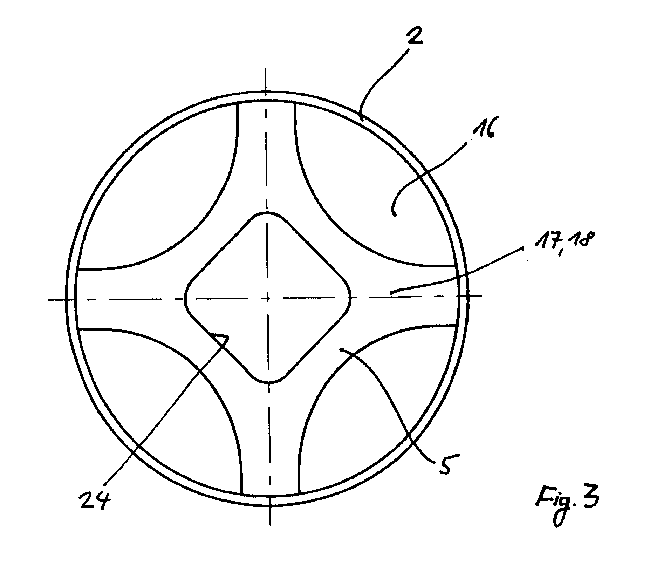

Abschließend sei auf die Aufsicht auf die Unterseite des chirurgischen Instrumentes 2 gemäß Figur 3 hingewiesen. Hier übergreifen Stege 17 einen Hohlraum 16 im Instrument 2. Die Stege 17 bilden hierdurch die Hinterschnitte 5, hinter welche die Kugeln 4 des Halters 1 im arretierten Zustand greifen und so für die sichere Befestigung des Instrumentes 2 am Halter 1 sorgen. Vorliegend sind die Stege 17 Bestandteil eines Doppelkreuzsteges 18. Durch die Öffnung 24 wird im dargestellten Ausführungsbeispiel das vordere Ende des Halters 1 geschoben, so weit daß die Kugeln 4 hinter die Stege 17 greifen.

## Patentansprüche

1. Kombination eines Halters (1) für ein chirurgisches Instrument (2), insbesondere für eine Raffelfräserklinge (2), mit einem chirurgischen Instrument (2) wobei der Halter (1) mit dem chirurgischen Instrument (2) durch einen Schnellverschluss (3) koppelbar ist, der im wesentlichen gebildet ist aus arretierbaren Kugeln (4), die im nichtarretierten Zustand unter Hinterschneidungen (5) am Instrument (2) gleiten und im arretierten Zustand hinter den Hinterschneidungen (5) verharren, **dadurch gekennzeichnet, dass** die Hinterschnitte (5) von, einen Hohlraum (16) im Instrument (2) übergreifenden Stegen (17) als Bestandteil eines Doppelkreuzsteges (8) gebildet sind.

2. Kombination nach Anspruch 1, **dadurch gekennzeichnet, dass** der Halter (1) im wesentlichen aus einer Hülse (6) besteht, in deren Inneren ein Dom (7) axial verschieblich gelagert ist, wobei der Dom (7) in vorgeschobenener Lage die Kugeln (4) in einem Käfiggehäuse (8) am distalen Ende (9), dem Koppelungsende des Halters (2), arretiert, wobei die Kugeln (4) über die Konturen des Käfiggehäuses (8) teilweise herausragen, und die Kugeln (4) in zurückgezogener Lage freigibt, damit diese in das Innere des Käfiggehäuses (8) fallen.

3. Kombination nach Anspruch 2, **dadurch gekennzeichnet, dass** der Halter (1) am rückseitigen Ende (10) mittels einer Buchse (11) verschließbar ist.

4. Kombination nach Anspruch 3, **dadurch gekennzeichnet, dass** zwischen dem Dorn (7) und der Buchse (11) eine Spiralfeder (12) eingesetzt ist, die den Dorn (7) in Richtung auf die vorgeschobene Lage vorspannt.

5. Kombination nach einem der Ansprüche 2 bis 4, **dadurch gekennzeichnet, dass** als ein Betätigungsorgan ein die Hülse (6) durch ein Langloch (13) hindurchgreifender Gewindebolzen (14) vorgesehen ist, der mit einer entsprechenden Gewindebohrung (15) im Dom (7) verschraubbar ist.

## Claims

1. Combination of a holder (1) for a surgical instrument (2), in particular for a reamer blade (2), and of a surgical instrument (2), the holder (1) being able to be coupled to the surgical instrument (2) by a quick-coupling means (3) which is principally formed by lockable balls (4) which, in the unlocked state, slide beneath undercuts (5) on the instrument (2) and, in the locked state, are immobilized behind the undercuts (5), **characterized in that** the undercuts (5) are formed by bridges (17) which engage over a cavity (16) in the instrument (2) and which are part of a double cross-bar (18).

2. Combination according to Claim 1, **characterized in that** the holder (1) consists principally of a sleeve (6) in the inside of which a rod (7) is mounted in an axially displaceable manner, which rod (7), in the advanced position, locks the balls (4) in a cage (8) at the distal end (9), the coupling end, of the holder (2), the balls (4) partially protruding beyond the contours of the cage (8), and, in the retracted position, releases the balls (4) so that these fall into the interior of the cage (8).

3. Combination according to Claim 2, **characterized in that** the holder (1) can be closed at the rear end (10) by means of a bushing (11).

4. Combination according to Claim 3, **characterized in that** between the rod (7) and the bushing (11) there is a helical spring (12) which pretensions the rod (7) in the direction of the advanced position.

5. Combination according to one of Claims 2 to 4, **characterized in that** an actuating element is provided in the form of a threaded bolt (14) which engages through an oblong hole (13) in the sleeve (6) and which can be screwed in a corresponding threaded bore (15) in the rod (7).

## Revendications

1. Combinaison.d'unsupport (1) pour uninstrument chirurgical(2), notamment pour unelamede fraise-râpe(2),avecun instrumentchirurgical (2), lesupport(1) pouvantêtreaccoupléà l'instrument chirurgical (2) par unefermeture rapide(3) qui estessentiellement formée à partir de billes blocables(4) qui,dansl'étatnon bloqué,glissentsousdes contre-dépouilles(5) surl'instrument(2),et dans l'état bloqué, s'immobilisent derrièreles contre- dépouilles(5), **caractérisée en ce que** les contre-dépouilles(5) sont formées par desailettes (17) venant en prise au-dessus d'un espacecreux (16) dans l'instrument (2) en tant que constituantdune âmeen double croix (18).

2. Combinaisonselon larevendication 1, caractérisée en ce quelesupport (1) secompose essentiellement d'unmanchon(6) àl'intérieur duquel estmontéunmandrin (7) demanièreà pouvoir coulisser axialement, le mandrin (7), dans la position pousséeen avant, bloquantles billes (4) dans un boîtier-cage (8) à l'extrémité distale (9), l'extrémitéd'accouplementdu support (2), tandis que les billes(4) sortentpartiellement au-delà des contoursduboîtier-cage (8) et libérant les billes (4) dans la position rentrée, afinquecelles-citombentàl'intérieurdu boîtier-cage (8).

3. Combinaison selon la revendication 2,**caractérisée en ce que** le support (1) peut être ferméà l'extrémité arrière (10) au moyen d'unedouille (11) .

4. Combinaisonselon la revendication 3, **caractérisée en ce que** l'on insère entre le mandrin (7)et la douille (11) un ressort spiral (12) qui précontraint le mandrin (7) dansla direction de la position poussée en avant.

5. Combinaisonselonl'unequelconque des revendications 2 à 4, **caractérisée en ce que** l'on prévoiten tant qu'organe d'actionnement un boulon fileté (14)venant en prise à travers le manchon (6) par le biais d'un trou oblong (13), lequel boulon fileté peut être vissé avec unalésage fileté correspondant (15) dans le mandrin (7).
